Europäisches Patentamt

European Patent Office

Office européen des brevets

(11) Veröffentlichungsnummer: **0 373 365 B1**

(12) **EUROPÄISCHE PATENTSCHRIFT**

(45) Veröffentlichungstag der Patentschrift: **18.05.94**

(51) Int. Cl.5: **C12N 15/70**, C12N 15/67, C12N 15/11, //C12N9/64, C12N15/58

(21) Anmeldenummer: **89120895.1**

(22) Anmeldetag: **10.11.89**

(54) Verfahren zur Expression eines rekombinanten Gens.

(30) Priorität: **11.11.88 DE 3838378**
**31.08.89 DE 3928899**
**25.09.89 DE 3931933**

(43) Veröffentlichungstag der Anmeldung:
**20.06.90 Patentblatt 90/25**

(45) Bekanntmachung des Hinweises auf die Patenterteilung:
**18.05.94 Patentblatt 94/20**

(84) Benannte Vertragsstaaten:
**AT BE CH DE ES FR GB GR IT LI LU NL SE**

(56) Entgegenhaltungen:
EP-A- 0 141 790
GB-A- 2 135 677

CELL, Band 45, 1986, Seiten 453-459, Cambridge, US; G.M. GARCIA et al.: "The E.coli dnaY gene encodes an arginine transfer RNA"

BIOLOGICAL ABSTRACTS, Band 83, Nr. 3, 1987, Zusammenfassung Nr. 79171, Philadelphia, US; A.K. ULRICH et al.: "Strains overproducing transfer RNA forhistidine"

(73) Patentinhaber: **BOEHRINGER MANNHEIM GMBH**

**D-68298 Mannheim(DE)**
(72) Erfinder: **Brinkmann, Ulrich, Dipl.-Biol.**
**Von-Siemens-Strasse 5**
**D-4700 Hamm(DE)**
Erfinder: **Mattes, Ralf**
**Friedrich-Zundel-Strasse 14**
**D-7000 Stuttgart 75(DE)**
Erfinder: **Fischer, Stephan, Dipl.-Biol.**
**Jakobifeldweg 11**
**D-8121 Polling(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**Beschreibung**

Die Erfindung betrifft ein Verfahren zur Expression eines rekombinanten Gens, das AGA- oder/und AGG-Kodons für Arginin enthält, in E. coli nach Transformation mit einem Expressionsvektor, der das rekombinante Gen enthält.

Die gentechnologische Herstellung von Proteinen oder proteinhaltigen Genprodukten ist eines der Hauptziele der modernen Biotechnologie. Da sich vor allem Prokaryonten aufgrund ihrer leichten Fermentierbarkeit zur Herstellung großer Proteinmengen eignen, wurde bereits vielfach versucht, auch eukaryontische Gene in Prokaryonten zu exprimieren. Hierbei ergeben sich jedoch Schwierigkeiten dahingehend, daß eukaryontische Proteine in Prokaryonten-Zellen oft nur in geringen Mengen hergestellt werden und die Zellen Fermentations/Wachstumsschwierigkeiten bei erhöhter Produktion der Proteine zeigen.

Derartige Probleme bei der Expression werden unter anderem bei dem Gewebs(tissue type)-Plasminogen-Aktivator t-PA beobachtet. Die Herstellung dieses Proteins ist jedoch ein erstrebenswertes Ziel, da es sich bei der klinischen Erprobung als zur Behandlung von Infarktkrankheiten geeignet erwiesen hat. Obwohl E. coli-Zellen die auf einem Plasmid oder Vektor eingeführte t-PA-cDNA in an sich befriedigender Menge exprimieren und t-PA produzieren können, lassen die Zellen jedoch bei der Bildung von Biomasse während der Fermentation stark nach, so daß insgesamt die Produktion von t-PA nicht den Stand erreicht, den man erwarten könnte, wenn man von der Produktion einer einzelnen Zelle ausgeht. Auch ist die Plasmidstabilität in den E. coli-Zellen gering und durch Verlust des kodierenden Plasmids nimmt die Produktionsrate weiter ab. Bisher ist es daher nur möglich, eine Expression von ca. 5 % des Gesamtzellproteins an t-PA zu erreichen (Rothstein und Bertonis, Gene 61 (1987), 41-50).

Der Erfindung lag nun die Aufgabe zugrunde, ein Verfahren bereitzustellen, das es ermöglicht, rekombinante Gene, deren Expression nach den bisher bekannten Verfahren zu einem deutlich verschlechterten Wachstum der Wirtszellen und Plasmidinstabilität und damit zu geringen Ausbeuten an Genprodukt führte, mit erhöhter Ausbeute zu exprimieren.

Gelöst wird die Aufgabe erfindungsgemäß durch ein Verfahren zur Expression eines rekombinanten Gens, das AGA- oder/und AGG-Kodons für Arginin enthält, in E. coli nach Transformation mit einem Expressionsvektor, der das rekombinante Gen enthält, das dadurch gekennzeichnet ist, daß man die in den E. coli-Zellen vorhandene Menge von t-RNA, die Arginin einbaut und die Kodons AGG und AGA erkennt, auf mindestens das Fünffache der normalerweise in diesen Zellen vorkommenden Menge erhöht.

Hierbei ist im Rahmen der Erfindung unter dem Begriff t-RNA, welche AGA/AGG-Kodons erkennt und Arginin einbaut, nicht nur die natürliche in E. coli vorkommende, entsprechende t-RNA zu verstehen, sondern auch jegliche synthetische, mutierte oder Suppressor-t-RNA, die die genannten Eigenschaften aufweist.

Das erfindungsgemäße Verfahren ermöglicht es, rekombinante Gene, deren Expression bisher aufgrund von schlechtem Wachstum der E. coli-Wirtszellen nur in geringen Ausbeuten zu den gewünschten Proteinen führte, in deutlich erhöhten Mengen herzustellen. Dieser erreichte technische Effekt ist insofern unerwartet, da bei einem Mangel an der erfindungsgemäß eingesetzten t-RNA bei der Proteinsynthese in den Zellen davon auszugehen gewesen wäre, daß das Fremdgen schlecht exprimiert würde, und nicht, wie tatsächlich der Fall ist, das Fremdgen gut exprimiert wird, aber die Zelle kümmert.

In einer bevorzugten Ausführungsform der Erfindung wird die Menge der t-RNA, die AGA/AGG-Kodons erkennt und Arginin einbaut (im weiteren auch nur mit t-RNA bezeichnet), in den E. coli-Zellen dadurch erhöht, daß man mindestens ein für eine derartige t-RNA kodierendes Gen in die Zelle einführt. Dies kann vorzugsweise dadurch geschehen, daß man ein oder mehrere t-RNA-Gene auf einem extrachromosomalen Expressionsvektor in die Zelle einführt.

Die natürliche t-RNA, welche in E. coli Arginin einbaut und die Kodons AGA und AGG erkennt, ist ein Produkt des dnaY-Gens, das von Garcia et al., in Cell 45 (1986), 453-459, beschrieben wurde. Dieses Gen ist in seiner gesamten Sequenz bekannt, es enthält 118 essentielle Basenpaare und wird bevorzugt zur Erhöhung der t-RNA-Menge in E. coli durch Einführung des Gens verwendet. Es ist jedoch auch möglich, ein Gen für eine synthetische, mutierte oder Suppressor-t-RNA, die Arginin einbaut und die AGA/AGG-Kodons erkennt, in die Zellen einzuführen. Ebenso kann das Gen der t-RNA aus dem Phagen $T_4$ verwendet werden.

In einer bevorzugten Ausführungsform der Erfindung bringt man das oder die t-RNA-Gene zusammen mit dem eukaryontischen Gen auf dem selben Expressionsvektor in die Zelle ein. Hierbei ergibt sich, wenn der Expressionsvektor ein High-copy-plasmid ist, bereits eine ausreichende Erhöhung des intrazellulären t-RNA-Spiegels, so daß keinerlei negative Auswirkungen auf die Wirtszellen bei der Expression des eukaryontischen Gens zu beobachten sind.

In einer anderen bevorzugten Ausführungsform bringt man das t-RNA-Gen und das eukaryontische Gen auf verschiedenen Expressionsvektoren in die E. coli-Zellen ein. Die positiven Auswirkungen der Bereitstellung einer t-RNA Arg(AGG/AGA) während der Expression rekombinanten Proteins in E. coli ist nämlich nicht davon abhängig, daß das Gen für diese t-RNA cis-ständig auf dem jeweiligen Expressionsvektor vorhanden ist. Diese t-RNA kann auch auf einem zweiten Vektor codiert sein (transaktiv). Dieser Zusatzvektor muß allerdings mit dem Expressionsvektor in der E. coli-Zelle kompatibel sein, d.h. einen von dem als Expressionsplasmid verschiedenen Replikationsursprung aufweisen.

Hierbei ist es möglich, einen Vektor mit geringerer Plasmidkopienzahl als das Expressionsplasmid zu benutzen, aber auch durch Verwendung eines Vektors, der in höherer Kopienzahl vorliegt als der Expressionsvektor, auf dem das eukaryontische Gen sich befindet, einen gegenüber dem eukaryontischen Gen noch höheren t-RNA-Spiegel zu erreichen. Im Falle des Einbringens des t-RNA-Gens und des eukaryontischen Gens auf dem selben Expressionsvektor, ist es sowohl möglich, die beiden Gene unter der Kontrolle von verschiedenen Promotoren, oder aber die beiden Gene in Form eines Operons unter der Kontrolle desselben Promotors einzusetzen. Wesentlich ist hierbei nur, daß sich zwischen dem eukaryontischen Gen und dem t-RNA-Gen ein Stopcodon befindet, so daß sich bei der Transkription kein Fusionsprotein ergibt.

In einer anderen bevorzugten Ausführungsform der Erfindung integriert man das oder die t-RNA-Gene in das Chromosom der Wirtszelle. Hierbei ist es besonders bevorzugt, das Gen derart zu integrieren, daß es unter einem starken Promotor exprimiert wird. Hierzu wird vorzugsweise das oder die t-RNA-Gene gleich zusammen mit einem starken Promotor, unter dessen Kontrolle die t-RNA exprimiert wird, eingeführt.

Zur Einführung eines t-RNA-Gens in das Chromosom werden ebenso wie zur Einführung des Gens in einen Expressionsvektor, sowie für die Transformation der Wirtszellen an sich bekannte Methoden angewandt. Zur Einführung in das Chromosom werden vorzugsweise Methoden unter Verwendung von Transposons oder Phagen und unter Ausnutzung von Rekombinationsereignissen angewandt.

Eine weitere bevorzugte Möglichkeit, die t-RNA-Menge in der Zelle zu erhöhen liegt darin, daß man den natürlichen Promotor des chromosomalen Gens, das für diese t-RNA kodiert, derart verändert, daß die t-RNA in größerer Menge gebildet wird. Hierzu wird besonders bevorzugt der gesamte natürliche Promotor gegen einen stärkeren, vorzugsweise induzier- oder/und reprimierbaren Promotor ausgetauscht. Derartige Promotoren sind dem Fachmann bekannt, ebenso die Verfahren zum Austausch der Promotoren, die wiederum vorzugsweise unter Verwendung von Transposons oder Phagen durchgeführt werden.

Ein weiterer Gegenstand der Erfindung ist ein Verfahren zur Expression eines rekombinanten Gens, das AGA-oder/und AGG-Kodons für Arginin enthält, in E. coli durch Transformation mit einem Expressionsvektor, der das rekombinante Gen enthält, das dadurch gekennzeichnet ist, daß man einen E. coli-Stamm selektioniert, der mindestens die fünffache Menge der normalerweise in E. coli vorkommenden Menge an t-RNA, die die Kodons AGG und AGA erkennt und Arginin einbaut, enthält und diesen Stamm als Wirtszellen verwendet.

Bisher bekannte Stämme weisen meist nur eine derart geringe Menge an t-RNA, die Arginin einbaut und die Kodons AGA/AGG erkennt, auf und es treten die in der Einleitung beschriebenen Folgen bei Expression eines Fremdgens ein. Als Standard für E. coli-Stämme mit der normalen geringen Menge der erfindungsgemäß eingesetzten t-RNA können beispielsweise die Stämme DSM 3689, DSM 2102, HFR 3000 und C600 (DSM 2093) (die beiden letzteren sind bei Bachmann, Bacteriol. Rev. 36 (1972), 180-230 beschrieben) aufgeführt werden. Gemäß dem erfindungsgemäßen Verfahren können nun gegebenenfalls E. coli-Stämme aufgefunden werden können, welche von Haus aus eine höhere, nämlich mindestens die doppelte Menge dieser t-RNA enthalten. Hierzu selektioniert man bevorzugt derart, daß man Gesamt-RNA der E. coli-Zellen herstellt und diese gegen ein markiertes Oligonukleotid hybridisiert, das einem spezifischen Teil der DNA-Sequenz der t-RNA entspricht. Bevorzugt wird daher die Northernblot-Technik verwendet. Die Northernblot-Technik ist dem Fachmann bekannt und beispielsweise in Maniatis et al., (1982), Molecular Cloning. A Laboratory Manual. Cold Spring Harbor Laboratory, Cold Spring Harbor, New York, beschrieben. Zur Hybridisierung verwendet man vorzugsweise ein radioaktiv markiertes Oligonukleotid, jedoch können auch alle anderen Markierungen für DNA-Fragmente vorteilhaft verwendet werden. Unter einem spezifischen Teil der DNA-Sequenz der t-RNA, die die Kodons AGG oder/und AGA erkennt, versteht man die DNA-Sequenzen am und in der direkten Nachbarschaft des Anticodons in der tRNA, der sogenannten Anticodon-Schleife. Im Gegensatz zu der Pseudouridin- oder Dihydrouridin-Schleife ist dieser Bereich einer tRNA nicht sehr homolog zu anderen tRNAs, so daß ein Oligonukleotid der Sequenz
5'- C A C G A C T T A G A A G G T C G T T G -3' oder auch z.B. der kürzeren Sequenz
5'- G A C T T A G A A G G T C G T T -3', sowie die jeweils komplementären Oligonukleotide
(5'- C A A C G A C C T T C T A A G T C G T G -3'),
(5'- A A C G A C C T T C T A A G T C -3') als spezifische Sonde zur Detektion und Charakterisierung dieser tRNA verwendet werden können. Erfindungsgemäß verwendet man vorzugsweise ein Oligonukleotid, das zwischen 14 und 30 Basenpaare lang ist.

Das erfindungsgemäße Verfahren eignet sich für alle rekombinanten Gene, vorzugsweise werden eukaryontische Gene bzw. deren cDNA exprimiert, und besonders solche, die eine größere Anzahl von AGG oder/und AGA-Kodons enthalten. Dies sind z.B. menschliche Urokinase, t-PA oder Derivate davon, HIV-Proteine (z.B. gp41, p24), α-Glucosidase aus Hefe. Ein großer Vorteil des erfindungsgemäßen Verfahrens liegt hierbei auch darin, daß die rekombinanten Gene konstitutiv exprimiert werden können.

Besonders bevorzugt ist das erfindungsgemäße Verfahren zur Herstellung von t-PA, indem man als rekombinantes Gen das t-PA-Gen (beschrieben von Pennica et al., Nature 301 (1983) 214-221) oder eine Variante davon exprimiert. Gegenüber der bisher im Stand der Technik bekannten Expressionsrate von etwa 5 % t-PA pro E. coli Gesamtzellprotein kann erfindungsgemäß die Ausbeute mehr als 30 % gesteigert werden. Dies bedeutet eine 600%ige Steigerung der Ausbeute mit Hilfe des erfindungsgemäßen Verfahrens.

In einer weiteren bevorzugten Ausführungsform der Erfindung exprimiert man das Plasmid pUBS 98.sky1, DSM 4898, in E. coli, das das Gen für t-PA und das dnaY-Gen enthält.

Weitere Gegenstände der Erfindung sind Expressionsvektoren, die ein rekombinantes Gen und ein Gen für eine t-RNA, die Arginin einbaut und die Kodons AGG oder/und AGA erkennt, enthalten. Hierbei können beide Gene jeweils unter der Kontrolle von verschiedenen Promotoren stehen, wobei die für das jeweiligen Gen homologen oder aber auch heterologe Promotoren verwendbar sind, oder aber, gemäß einer bevorzugten Ausführungsform der Erfindung, unter der Kontrolle eines gemeinsamen Promotors in Form eines Operons stehen.

Ein allgemein zur Klonierung und Expression von rekombinanten Genen in E. coli verwendbarer bevorzugter Expressionsvektor besteht aus

- einem konstitutiven oder regulierbaren in E. coli transkribierbaren Promotor (z.B. lac-, tac-, trp-, E. coli ribosomale Promotoren, Bacillus stearothermophilus α-Galactosidase-Promotor),
- wahlweise gefolgt von einer Ribosomenbindestelle zur Translationsinitiation,
- wahlweise gefolgt von einem Translationsinitiationscodon (bevorzugt ATG)
- gefolgt von einem Polylinker zur Klonierung des zu exprimierenden Gens/cDNA,
- wahlweise gefolgt von einer mRNA-stabilisierenden Sequenz,
- gefolgt von einem Transcriptionsterminator (vorzugsweise fd-Terminator),
- einem Resistenzgen (vorzugsweise Kanamycinresistenzgen),
- einem in E. coli zur stabilen Plasmidpropagation fähigen Replikationsorigin,
- sowie einem Gen für eine tRNA Arg(AGG/AGA).

Ein erfindungsgemäß besonders bevorzugtes Plasmid ist pUBS 98.sky1, DSM 4898. Dieses Plasmid enthält sowohl das dnaY-Gen als auch das t-PA-Gen.

Ein weiterer Gegenstand der Erfindung sind Expressionshilfsvektoren, die das Gen für eine t-RNA, die Arginin einbaut und die Kodons AGG oder/und AGA erkennt, enthalten und einen von dem der ColE1-Plasmide und deren Abkömmlingen verschiedenen Replikationsursprung besitzen.

Hierdurch wird eine Cokultivierung zusammen mit einem ColEl-Expressionsvektor in E. coli-Zellen ermöglicht.

In einer bevorzugten Ausführungsform der Erfindung steht das Gen für die t-RNA unter der Kontrolle seines in E. coli natürlichen, also homologen Promotors, in einer anderen bevorzugten Ausführungsform unter der eines heterologen Promotors, besonders bevorzugt des lac-, tac-, mgl- oder trp-Promotors, oder des α-Galactosidase-Promotors aus Bacillus stearothermophilus.

Weitere Gegenstände der Erfindung sind die Verwendung der obigen Expressionsvektoren zur Expression des darin vorhandenen rekombinanten Gens, wobei stark erhöhte Ausbeuten erhalten werden, und die Verwendung der obengenannten Expressionshilfsvektoren zur Erhöhung der Ausbeute bei der Expression eines Gens, das in Form einer zur Expression des Gens geeigneten rekombinanten DNA in eine E. coli-Zelle eingebracht wird. Erfindungsgemäß läßt sich hierbei, bei Verwendung eines konstitutiven Promotors, das rekombinante Gen konstitutiv exprimieren.

Die folgenden Beispiele erläutern in Verbindung mit der Abbildung die Erfindung weiter.

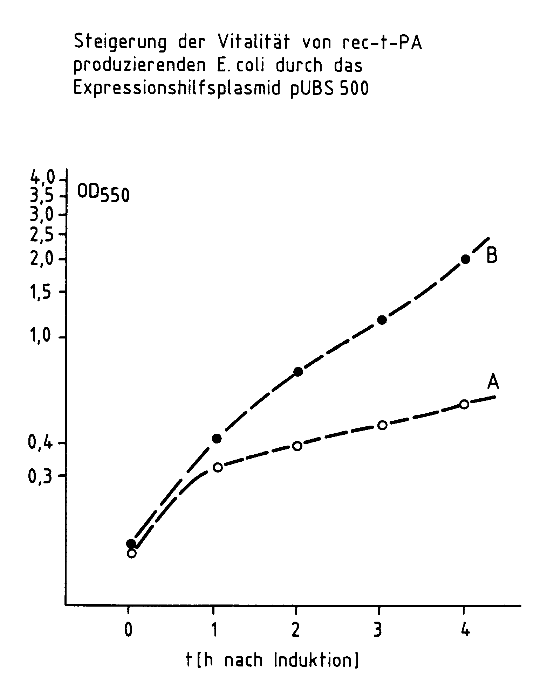

Fig. 1/Tabelle 1    zeigt die Steigerung der Expressionsleistung von rekombinantem t-PA bei Induktion und Cotransfektion mit einem Expressionshilfsplasmid, sowie die positiven Auswirkungen des Expressionshilfsplasmides auf die Vitalität der Zellen bei t-PA-Expression.

**Beispiel 1**

Konstruktion von t-PA-Expressionsplasmiden

a) Plasmid pePa 126.1

Als Ausgangsplasmid zur Konstruktion eines verbesserten t-PA-Expressionsplasmids wurde das Plasmid pePa 98.1 (EP-A 242 836) verwendet. Die ca. 400 bp lange 3'-untranslatierte Region der t-PA-cDNA in diesem Plasmid wurde durch Deletion eines 361 bp langen XhoII-Fragmentes auf ca. 40 bp verkürzt.

Das resultierende Plasmid erhielt den Namen pePa 126.1, und kann von pePa 98.1 z.B. dadurch unterschieden werden, daß bei einem Doppelverdau dieser Plasmide mit den Restriktionsendonukleasen BamHI und HindIII bei pePa 98.1 zwei Fragmente der Länge 2234 bp und 4372 bp nachgewiesen werden können, bei Plasmid pePA 126.1 hingegen zwei Fragmente der Länge 1873 bp und 4372 bp.

b) Plasmid pUBS 98.skyI

Das Expressionsplasmid pUBS 98.skyI enthält ein ca. 3000 bp großes DraI-Fragment von Plasmid pDM 201, welches ein Gen (dnaY) für eine t-RNA Arg(AGG/AGA) enthält (Garcia et al., Cell 45, 1986, 453-459), das in ein DraI geschnittenes t-PA-Expressionsplasmid insertiert wurde, welches ein kanamycinresistentes Derivat des oben beschriebenen Plasmids pePa 126.1 ist. pUBS 98.sky1 wurde unter der Nummer 4898 bei der Deutschen Sammlung für Mikroorganismen (DSM) hinterlegt.

**Beispiel 2**

Expression von t-PA in E. coli mit einem Expressionshilfeplasmid.

Das Gen für die t-RNA Arg(AGG/AGA) wurde auf ein Plasmid kloniert, welches kompatibel ist mit dem t-PA-Expressionsvektor pePa 126.1, welcher einen ColE1-Replikationsorigin besitzt (ein pBR322-Derivat ist).

Plasmid pACYC 177 (Chang und Cohen, J. Bact. 134, 1978, 1141-1156), DSM 3693P, wurde hierzu mit DraI geschnitten und ein 3230 bp-Fragment isoliert. Dieses Fragment wurde mit dem ca. 3000 bp DraI-Fragment aus Plasmid pDM 201, welches das dnaY-Gen enthält ligiert, und der Ligationsansatz in E. coli, DSM 2102, transformiert.

Kanamycinresistente Klone, die das Plasmid pUBS 400 bzw. pUBS 401 enthalten, wurden durch Koloniefilterhybridisation mit dem Oligonukleotid [5'- A G C A A C G A C C T T C T A A G T C G T G G G -3'] identifiziert und isoliert. Die derart isolierten Plasmide pUBS 400 und pUBS 401 unterscheiden sich von pACYC 177 durch ein 3000 bp Insert in der DraI-Schnittstelle, auf welchem sich das Gen für eine t-RNA Arg(AGG/AGA) befindet, und untereinander in der Orientierung des 3000 bp-DraI-Inserts (Beispiel 1) im Vektor.

pUBS 400 wird dadurch charakterisiert, daß in Southernanalysen HindII-HindIII-doppelverdauter Plasmid-DNA ein ca. 3300 bp-Fragment mit dem synthetischen Oligonukleotid [5'- A G C A A C G A C C T T C T A A G T C G T G G G -3'] hybridisiert. pUBS 401 wird dadurch charakterisiert, daß in analog durchgeführten Analysen ein ca. 1700 bp-Fragment hybridisiert.

Das Plasmid pIQ 500 ist ein pACYC 177-Derivat, welches das lac I$^q$-Gen enthält. Das Plasmid pIQ 500 enthält das lac I-Gen (P.J. Farabaugh, Nature 274, 765-769, 1978) mit der iq-Promotormutation (Calos, Nature 274, 762-765, 1978) als HindII-Fragment (partial aus pMCI Calos 1978 s.o.) insertiert in HindII-geschnittenes Plasmid pACYC 177 (Chang and Cohen J. Bact. 134, 1978, 1141-1156).

pIQ 500 enthaltende E. coli-Zellen sind kanamycinresistent und (- im Gegensatz zu pACYC 177 enthaltenden) ampicillinsensibel, und enthalten eine wesentlich höhere Konzentration an Lac-Repressormolekülen (lacI) als vergleichbare E. coli ohne dieses Plasmid. pIQ 500 wurde zur Unterstützung der Repression rekombinanter Gene im nicht induzierten Zustand verwendet, sofern diese Gene unter Kontrolle des lac-Promotors oder Derivaten davon, z.B. tac; trc usw. transkribiert werden, und die Expressionsplasmide kompatibel mit pIQ 500 sind (wie z.B. pKK 223-3-Derivate).

Die Klonierung eines t-RNA-Arg(AGG/AGA)-Gens auf dieses Plasmid zusätzlich zum lac Iq-Gen ermöglicht es, der E.coli-Zelle nicht nur die zur Repression im nicht induzierten Zustand erforderlichen Mengen an lac-Repressor, sondern auch die während der Expression eukaryontischer Proteine benötigten großen Mengen an t-RNA Arg(AGG/AGA) zu liefern.

Plasmid pIQ 500 wurde mit DraI geschnitten, und ein 4836 bp Fragment isoliert. Dieses Fragment wurde mit dem ca. 3000 bp DraI-Fragment aus Plasmid pDM 201, welches das dnaY-Gen enthält ligiert, und der Ligationsansatz in E. coli, DSM 2102, transformiert.

Kanamycinresistente Klone, welche das Plasmid pUBS 500 bzw. pUBS 501 enthalten, wurden durch Koloniefilterhybridisation mit dem synthetisch hergestellten Oligonukleotid [5'- A G C A A C G A C C T T C T A A G T C G T G G G -3'] identifiziert und isoliert.

Die derart isolierten Plasmide pUBS 500 und pUBS 501 unterscheiden sich von pIQ 500 durch ein 3000 bp Insert in der DraI-Schnittstelle, auf welchem sich das Gen für eine t-RNA Arg(AGG/AGA) befindet, und untereinander in der Orientierung des 3000 bp-Inserts im Vektor. pUBS 500 wird dadurch charakterisiert, daß in Southernanalysen HindII-verdauter Plasmid-DNA ein ca. 4000 bp-Fragment mit dem synthetischen Oligonukleotid [5′- A G C A A C G A C C T T C T A A G T C G T G G G -3′] hybridisiert. pUBS 501 wird dadurch charakterisiert, daß in analog durchgeführten Analysen ein ca. 1700 bp-Fragment hybridisiert.

Die Expressionshilfeplasmide pUBS 400 und pUBS 500 sind in der Lage, transaktiv die negativen Auswirkungen der t-PA-Expression mit dem Plasmid pePa 126.1 durch Bereitstellung der nötigen t-RNA Arg(AGG/AGA) zu kompensieren.

Expression bei Cotransformation von gängigen E. coli-Laborstämmen (z.B. C-600) mit Plasmid pUBS 400 oder pUBS 500 und pePa 126.1 führt zu Ausbeuten und Vitalität wie bei t-PA-Produktion mit pUBS 98.sky1.

Tabelle 1 und Fig. 1 zeigen den Vergleich der Expression von rekombinanten t-PA aus pePa 126.1 in E. coli, DSM 2102, cotransformiert mit pIQ 500 (Probe A) bzw. pUBS 500 (Probe B) bei Induktion mit 5 mM IPTG.

Tabelle 1

| Probe | E. coli Plasmid | % Rec. Protein (intakter t-PA) Gesamtprotein | OD 550 bei Induktion | OD 550 3 Std. nach Induktion |
|---|---|---|---|---|
| A | E. coli, DSM 2102 + pePa 126.1 + pIQ 500 | 2-5 | 0,1 | 0,4 |
| B | E. coli DSM 2102 + pePa 126.1 + pUBS 500 | >30 | 0,1 | 1,2 |

**Beispiel 3**

Vergleich der Expression von t-PA in E. coli

Die Plasmide pePA 133 (DE 36 13 401), pePA 126.1 (Beispiel 1) und pUBS 98.sky1, DSM 4898, wurden in E. coli, DSM 3689, transformiert, der ein I$^q$-Plasmid enthält. Transformanten wurden auf LB-Platten (Maniatis, (1982) supra) mit 25 $\mu$g/ml Kanamycin (bei pePa 133 und pUBS 98.sky1) und 50 $\mu$g/ml Ampicillin (bei pePa 126.1) angezogen.

Plasmidhaltige Zellen wurden in LB bis zur OD 550 nm = 0,3 angezogen, induziert (Zusatz von 10 mmol/l IPTG) und bei 37°C fermentiert. Das Wachstum der Kulturen wurde durch Kontrolle der Zelldichte (OD 550) in regelmäßigen Abständen kontrolliert.

4 Stunden nach Induktion wurden die Zellen geerntet und durch Ultraschallbehandlung aufgeschlossen. Das so gewonnene Zell-Lysat wurde elektrophoretisch analysiert (Coomassieblau gefärbte SDS-Gele), t-PA wurde in Westernblots mit polyklonalen Antikörpern gegen t-PA aus Ziege nachgewiesen und über densitometrische Auswertung coomassiegefärbter SDS-Gele nach vorhergegangener Proteinbestimmung des Zellysates quantifiziert.

Tabelle 2 zeigt einen Vergleich der Ausbeuten und anderer Parameter bei der Expression der genannten Plasmide in E. coli, DSM 3689.

Tabelle 2

| Plasmid | t-PA qualit. | Ausbeute intakt | Vitalität | Plasmidstabilität |
|---|---|---|---|---|
| pePA 133.6 | intakt | 3-5% | gut | n.d. |
| pePA 126.1 | Fragmente | 5% | schlecht | schlecht |
| pUBS 98.sky1 | intakt | >30% | gut | gut |

Die t-PA-Expression mit Plasmid pePa 133.6 ist schon in der DE-36 13 401 beschrieben.

Die Expression mit Plasmid pePa 126.1 beeinträchtigt das Wachstum produzierender E. coli in 5 ml Rollerkulturen nur unwesentlich, wesentlich jedoch im Fermentationsmaßstab (≧10 l), und außerdem wird intakter t-PA nur in geringer Menge produziert, statt dessen t-PA-Fragmente in großer Menge.

Expression von t-PA in E. coli mit Plasmid pUBS 98.sky1 ermöglicht eine sehr hohe Expressionsleistung intakten t-PA's (30 % des Gesamtzellproteins), ohne negative Auswirkungen auf den Produktionsorganismus. t-PA produzierende Zellen wachsen mit diesem Plasmid sehr schnell und zu hohen Zelldichten, t-PA läßt sich sogar konstitutiv exprimieren.

**Beispiel 4**

Vergleich der Expression von verschiedenen Proteinen in E. coli mit und ohne Expressionshilfsplasmid

Die Plasmide pUPA 110 (enthaltend die Sequenz für menschliche Pro-Urokinase ohne Signalpeptid, Holmes et al., Biotechnology 3 (1985) 923-929), pGP41 (enthaltend die codierende Sequenz für das p41-Protein des menschlichen HIV-Virus, Ratner et al., Nature 313 (1985) 277-284), pKK 177-3/GLUCPI (enthaltend die Sequenz für α-Glucosidase aus Hefe, EPA 0 300 425), pBT 102 (DSM 2091) (α-Glucosidase aus E. coli) und Plasmid pUR 289 (enthaltend die Sequenz für β-Galactosidase aus E. coli, Rüther und Mulker, EMBO J. 2 (1983) 1791-1794) wurden in E. coli, DSM 3689, der ein I^q-Plasmid enthält, transformiert. Die Anzucht der Transformanten sowie die Fermentation wurden analog Beispiel 3 durchgeführt.

Tabelle 3 zeigt den Vergleich der Expression der rekombinanten Proteine mit und ohne das Expressionshilfsplasmid pUBS 500.

Tabelle 3

| 1. Plasmid | 2. Plasmid | % rekombinantes Protein, bezogen auf Gesamtprotein-menge | OD 550 bei Induktion | OD 550 bei Ernte | Vitalität der Zellen bei Expression |
|---|---|---|---|---|---|
| pUPA 110 | piq 500[2] | 7 % | 0,15 | 0,70 | (-) |
| pUPA 110 | pUBS 500 | >30 % | 0,15 | 1,30 | + |
| pGP 41 | piq 500 | <2 % | 0,10 | 0,20 | - |
| pGP 41 | pUBS 500 | >30 % | 0,10 | 0,90 | + |
| pKK 177-3 | piq 500 | 15 % | 0,15 | n.d.[1] | (+) |
| pKK 177-3 | pUBS 500 | 30 % | 0,15 | n.d. | + |
| pUR 289 | piq 500 | 20 % | 0,15 | n.d. | (+) |
| pUR 289 | pUBS 500 | 30 % | 0,15 | n.d. | + |

n.d.[1]: nicht gemessen

2) piq 500 ist das Basisplasmid von pUBS 500 ohne t-RNA-Arg(AGG/AGA)-Gen

Es zeigt sich, daß bei Expression von Pro-Urokinase und HIV-GP 41, deren mRNA einen sehr hohen AGG- und AGA-Gehalt aufweist, bei Expression ohne Expressionshilfsplasmid die Expressionsausbeute und Vitalität der exprimierenden E. coli-Zellen deutlich reduziert ist. Durch Zugabe des Expressionshilfsplasmids pUBS 500 wird eine sehr hohe Expressionsleistung sowie eine hohe Vitalität der Klone erreicht.

Auch die Expression der α-Glucosidase wird durch das Expressionshilfsplasmid gesteigert. Da dieses Gen jedoch weniger AGG/AGA-Codons beinhaltet als Pro-Urokinase und HIV-GP 41, ist auch die beobachtete Expressionssteigerung bei Zugabe des Expressionshilfsplasmids nicht so drastisch wie für Pro-Urokinase und HIV-GPG 1.

Das Gen für die α-Galactosidase aus E. coli beinhaltet noch weniger AGG/AGA-Codons als das α-Glucosidase-Gen aus Hefe. Deshalb ist die Steigerung der Expression durch Zugabe des Expressionshilfsplasmids relativ gering, aber immer noch deutlich sichtbar.

**Patentansprüche**

**Patentansprüche für folgende Vertragsstaaten : AT, BE, CH, DE, FR, GB, GR, IT, LI, LU, NL, SE**

1. Verfahren zur Expression eines rekombinanten Gens, das AGA- oder/und AGG-Kodons für Arginin enthält, in E. coli nach Transformation mit einem Expressionsvektor, der das rekombinante Gen enthält, **dadurch gekennzeichnet,** daß man die in den E. coli Zellen vorhandene Menge von t-RNA, die Arginin einbaut und die Kodons AGG und AGA erkennt, auf mindestens das Fünffache der normalerweise in diesen Zellen vorkommenden Menge erhöht.

2. Verfahren nach Anspruch 1, **dadurch gekennzeichnet,** daß man mindestens ein für diese t-RNA kodierendes Gen in die Zelle einführt.

3. Verfahren nach Anspruch 1, **dadurch gekennzeichnet,** daß man den natürlichen Promotor des chromosomalen Gens, das für diese t-RNA kodiert, derart verändert, daß die t-RNA in größerer Menge gebildet wird.

4. Verfahren nach Anspruch 1, **dadurch gekennzeichnet,** daß man den natürlichen Promotor des chromosomalen Gens, das für diese t-RNA kodiert, gegen einen stärkeren, vorzugsweise induzier- oder/und reprimierbaren Promotor austauscht.

5. Verfahren zur Expression eines rekombinanten Gens, das AGA- oder/und AGG-Kodons für Arginin enthält, in E. coli durch Transformation mit einem Expressionsvektor, der das rekombinante Gen enthält, **dadurch gekennzeichnet,** daß man einen Stamm selektioniert, der mindestens die fünffache Menge der normalerweise in E. coli vorkommenden Menge an t-RNA, die die Kodons AGG und AGA erkennt und Arginin einbaut, enthält und diesen Stamm als Wirtszellen verwendet.

6. Verfahren nach Anspruch 5, **dadurch gekennzeichnet,** daß man selektioniert, indem man Gesamt-RNA der E. coli-Zellen isoliert und diese gegen ein markiertes Oligonukleotid hybridisiert, das einem spezifischen Teil der DNA-Sequenz der t-RNA entspricht.

7. Expressionsvektor, **dadurch gekennzeichnet,** daß er ein rekombinantes Gen und ein Gen für eine t-RNA, die Arginin einbaut und die Kodons AGG oder/und AGA erkennt, enthält.

8. Plasmid pUBS 98 sky.1, DSM 4898.

9. Expressionshilfsvektor, **dadurch gekennzeichnet,** daß er das Gen für eine t-RNA, die Arginin einbaut und die Kodons AGG oder/und AGA erkennt, enthält und exprimiert und kompatibel zu einem Expressionsvektor mit einem ColE1-Replikationsursprung ist.

10. Verwendung eines Expressionsvektors nach einem der Ansprüche 7 oder 8 zur Expression des rekombinanten Gens in E. coli.

11. Verwendung eines Expressionshilfsvektors, der das Gen für eine t-RNA, die Arginin einbaut und die Kodons AGG oder/und AGA erkennt, enthält zur Erhöhung der Ausbeute bei der Expression eines rekombinanten Gens, das AGA- oder/und AGG-Kodons für Arginin enthält, in E. coli durch Cotransfektion von E. coli-Zellen mit dem Expressionshilfsvektor und einem, das rekombinante Gen enthaltenden Vektor, wobei der Expressionshilfsvektor einen von dem das rekombinante Gen enthaltenden Vektor verschiedenen Replikationsursprung aufweist.

# EP 0 373 365 B1

**Patentansprüche für folgenden Vertragsstaat : ES**

1. Verfahren zur Expression eines rekombinanten Gens, das AGA- oder/und AGG-Kodons für Arginin enthält, in E. coli nach Transformation mit einem Expressionsvektor, der das rekombinante Gen enthält, **dadurch gekennzeichnet,** daß man die in den E. coli Zellen vorhandene Menge von t-RNA, die Arginin einbaut und die Kodons AGG und AGA erkennt, auf mindestens das Fünffache der normalerweise in diesen Zellen vorkommenden Menge erhöht.

2. Verfahren nach Anspruch 1, **dadurch gekennzeichnet,** daß man mindestens ein für diese t-RNA kodierendes Gen in die Zelle einführt.

3. Verfahren nach Anspruch 1, **dadurch gekennzeichnet,** daß man den natürlichen Promotor des chromosomalen Gens, das für diese t-RNA kodiert, derart verändert, daß die t-RNA in größerer Menge gebildet wird.

4. Verfahren nach Anspruch 1, **dadurch gekennzeichnet,** daß man den natürlichen Promotor des chromosomalen Gens, das für diese t-RNA kodiert, gegen einen stärkeren, vorzugsweise induzier- oder/und reprimierbaren Promotor austauscht.

5. Verfahren zur Expression eines rekombinanten Gens, das AGA- oder/und AGG-Kodons für Arginin enthält, in E. coli durch Transformation mit einem Expressionsvektor, der das rekombinante Gen enthält, **dadurch gekennzeichnet,** daß man einen Stamm selektioniert, der mindestens die fünffache Menge der normalerweise in E. coli vorkommenden Menge an t-RNA, die die Kodons AGG und AGA erkennt und Arginin einbaut, enthält und diesen Stamm als Wirtszellen verwendet.

6. Verfahren nach Anspruch 5, **dadurch gekennzeichnet,** daß man selektioniert, indem man Gesamt-RNA der E. coli-Zellen isoliert und diese gegen ein markiertes Oligonukleotid hybridisiert, das einem spezifischen Teil der DNA-Sequenz der t-RNA entspricht.

7. Verfahren nach Anspruch 1, **dadurch gekennzeichnet,** daß man das Plasmid pUBS 98 sky.1, DSM 4898 als Expressionsvektor verwendet.

8. Verfahren nach Anspruch 7, **dadurch gekennzeichnet,** daß man die Expression des rekombinanten Gens in E. coli vornimmt.

9. Verwendung eines Expressionshilfsvektors, der das Gen für eine t-RNA, die Arginin einbaut und die Kodons AGG oder/und AGA erkennt, enthält zur Erhöhung der Ausbeute bei der Expression eines rekombinanten Gens, das AGA- oder/und AGG-Kodons für Arginin enthält, in E. coli durch Cotransfektion von E. coli-Zellen mit dem Expressionshilfsvektor und einem, das rekombinante Gen enthaltenden Vektor, wobei der Expressionshilfsvektor einen von dem das rekombinante Gen enthaltenden Vektor verschiedenen Replikationsursprung aufweist.

**Claims**
**Claims for the following Contracting States : AT, BE, CH, DE, FR, GB, GR, IT, LI, LU, NL, SE**

1. Process for the expression of a recombinant gene, which contains AGA and/or AGG codons for arginine, in E. coli after transformation with an expression vector which contains the recombinant gene, characterised in that one increases the amount of t-RNA present in the E. coli cells which incorporates arginine and recognises the codons AGG and AGA to at least fivefold of the amount normally occurring in these cells.

2. Process according to claim 1, characterised in that one introduces at least one gene coding for this t-RNA into the cells.

3. Process according to claim 1, characterised in that one changes the natural promotor of the chromosomal gene which codes for this t-RNA in such a manner that the t-RNA is formed in larger amounts.

4. Process according to claim 1, characterised in that one exchanges the natural promotor of the chromosomal gene which codes for this t-RNA for a stronger, preferably inducable and/or repressable promotor.

5. Process for the expression in E. coli of a recombinant gene which contains AGA and/or AGG codons for arginine by transformation with an expression vector which contains the recombinant gene, characterised in that one selects a strain which contains at least the fivefold amount of the amount of t-RNA normally occurring in E. coli which recognises the codons AGG and AGA and incorporates arginine and uses this strain as host cells.

6. Process according to claim 5, characterised in that one selects and isolates whole RNA of the E. coli cells and hybridises this against a labelled oligonucleotide which corresponds to a specific part of the DNA sequence of the t-RNA.

7. Expression vector, characterised in that it contains a recombinant gene and a gene for a t-RNA which incorporates arginine and recognises the codons AGG and/or AGA.

8. Plasmid pUBS 98 sky1, DSM 4898.

9. Expression auxiliary vector, characterised in that it contains and expresses the gene for a t-RNA which incorporates arginine and recognises the codons AGG and/or AGA and is compatible to an expression vector with a ColE1 replication origin.

10. Use of an expression vector according to one of claims 7 to 8 for the expression of the recombinant gene in E. coli.

11. Use of an expression auxiliary vector which contains the gene for a t-RNA which incorporates arginine and recognises the codons AGG and/or AGA for the increase of the yield in the case of the expression of a recombinant gene which contains AGA and/or AGG codons for arginine in E. coli by co-transfection of E. coli cells with the expression auxiliary vector and a vector containing the recombinant gene, whereby the expression auxiliary vector has a replication origin different from the vector containing the recombinant gene.

**Claims for the following Contracting State : ES**

1. Process for the expression of a recombinant gene, which contains AGA and/or AGG codons for arginine, in E. coli after transformation with an expression vector which contains the recombinant gene, characterised in that one increases the amount of t-RNA present in the E. coli cells which incorporates arginine and recognises the codons AGG and AGA to at least fivefold of the amount normally occurring in these cells.

2. Process according to claim 1, characterised in that one introduces at least one gene coding for this t-RNA into the cells.

3. Process according to claim 1, characterised in that one changes the natural promotor of the chromosomal gene which codes for this t-RNA in such a manner that the t-RNA is formed in larger amounts.

4. Process according to claim 1, characterised in that one exchanges the natural promotor of the chromosomal gene which codes for this t-RNA for a stronger, preferably inducable and/or repressable promotor.

5. Process for the expression in E. coli of a recombinant gene which contains AGA and/or AGG codons for arginine by transformation with an expression vector which contains the recombinant gene, characterised in that one selects a strain which contains at least fivefold of the amount of t-RNA normally occurring in E. coli which recognises the codons AGG and AGA and incorporates arginine and uses this strain as host cells.

6. Process according to claim 5, characterised in that one selects and isolates whole RNA of the E. coli cells and hybridises this against a labelled oligonucleotide which corresponds to a specific part of the DNA sequence of the t-RNA.

7. Process according to claim 1, characterised in that one uses the plasmid pUBS 98.sky1, DSM 4898, as expression vector.

8. Process according to claim 7, characterised in that one carries out the expression of the recombinant gene in E. coli.

9. Use of an expression auxiliary vector which contains the gene for a t-RNA which incorporates arginine and recognises the codons AGG and/or AGA for the increase of the yield in the case of the expression of a recombinant gene which contains AGA and/or AGG codons for arginine in E. coli by co-transfection of E. coli cells with the expression auxiliary vector and a vector containing the recombinant gene, whereby the expression auxiliary vector has a replication origin different from the vector containing the recombinant gene.

**Revendications**
**Revendications pour les Etats contractants suivants : AT, BE, CH, DE, FR, GB, GR, IT, LI, LU, NL, SE**

1. Procédé d'expression d'un gène recombinant qui contient le codon AGA et/ou AGG pour l'arginine, dans E. coli après transformation avec un vecteur d'expression qui contient le gène recombinant, caractérisé en ce que l'on augmente la quantité, présente dans les cellules de E. coli, d'ARNt qui incorpore l'arginine et reconnaît les codons AGG et AGA au moins à cinq fois la quantité normalement présente dans ces cellules.

2. Procédé selon la revendication 1, caractérisé en ce que l'on introduit dans la cellule au moins un gène codant cet ARNt.

3. Procédé selon la revendication 1, caractérisé en ce que l'on modifie le promoteur naturel du gène chromosomique qui code cet ARNt de façon que l'ARNt soit formé en quantité plus importante.

4. Procédé selon la revendication 1, caractérisé en ce que l'on remplace le promoteur naturel du gène chromosomique qui code cet ARNt par un promoteur plus puissant, de préférence inductible et/ou réprimable.

5. Procédé d'expression d'un gène recombinant qui contient le codon AGA et/ou AGG pour l'arginine, dans E. coli par transformation avec un vecteur d'expression qui contient le gène recombinant, caractérisé en ce que l'on sélectionne une souche qui contient une quantité au moins égale à cinq fois la quantité normalement présente dans E. coli d'ARNt qui reconnaît les codons AGG et AGA et qui incorpore l'arginine et l'on utilise cette souche comme cellules hôtes.

6. Procédé selon la revendication 5, caractérisé en ce que l'on sélectionne en isolant l'ARN total des cellules de E. coli et en l'hybridant à un oligonucléotide marqué qui correspond à une partie spécifique de la séquence d'ADN de l'ARNt.

7. Vecteur d'expression caractérisé en ce qu'il contient un gène recombinant et un gène d'un ARNt qui incorpore l'arginine et reconnaît les codons AGG et/ou AGA.

8. Plasmide pUBS 98 sky.1, DSM 4898.

9. Vecteur d'expression auxiliaire caractérisé en ce qu'il contient et exprime le gène d'un ARNt qui incorpore l'arginine et reconnaît les codons AGG et/ou AGA et est compatible avec un vecteur d'expression comportant une origine de réplication de ColE1.

10. Utilisation d'un vecteur d'expression selon l'une des revendications 7 ou 8 pour l'expression du gène recombinant dans E. coli.

**11.** Utilisation d'un vecteur d'expression auxiliaire qui contient le gène d'un ARNt qui incorpore l'arginine et reconnaît les codons AGG et/ou AGA pour augmenter le rendement lors de l'expression d'un gène recombinant qui contient le codon AGA et/ou AGG pour l'arginine, dans E. coli par cotransfection de cellules de E. coli avec le vecteur d'expression auxiliaire et un vecteur contenant le gène recombinant, le vecteur d'expression auxiliaire présentant une origine de réplication différente de celle du vecteur contenant le gène recombinant.

**Revendications pour l'Etat contractant suivant : ES**

**1.** Procédé d'expression d'un gène recombinant qui contient le codon AGA et/ou AGG pour l'arginine, dans E. coli après transformation avec un vecteur d'expression qui contient le gène recombinant, caractérisé en ce que l'on augmente la quantité, présente dans les cellules de E. coli, d'ARNt qui incorpore l'arginine et reconnaît les codons AGG et AGA au moins à cinq fois la quantité normalement présente dans ces cellules.

**2.** Procédé selon la revendication 1, caractérisé en ce que l'on introduit dans la cellule au moins un gène codant cet ARNt.

**3.** Procédé selon la revendication 1, caractérisé en ce que l'on modifie le promoteur naturel du gène chromosomique qui code cet ARNt de façon que l'ARNt soit formé en quantité plus importante.

**4.** Procédé selon la revendication 1, caractérisé en ce que l'on remplace le promoteur naturel du gène chromosomique qui code cet ARNt par un promoteur plus puissant, de préférence inductible et/ou réprimable.

**5.** Procédé d'expression d'un gène recombinant qui contient le codon AGA et/ou AGG pour l'arginine, dans E. coli par transformation avec un vecteur d'expression qui contient le gène recombinant, caractérisé en ce que l'on sélectionne une souche qui contient une quantité au moins égale à cinq fois la quantité normalement présente dans E. coli d'ARNt qui reconnaît les codons AGG et AGA et qui incorpore l'arginine et l'on utilise cette souche comme cellules hôtes.

**6.** Procédé selon la revendication 5, caractérisé en ce que l'on sélectionne en isolant l'ARN total des cellules de E. coli et en l'hybridant à un oligonucléotide marqué qui correspond à une partie spécifique de la séquence d'ADN de l'ARNt.

**7.** Procédé selon la revendication 1, caractérisé en ce que l'on utilise le plasmide pUBS 98 sky.1, DSM 4898 comme vecteur d'expression.

**8.** Procédé selon la revendication 7, caractérisé en ce que l'on réalise l'expression du gène recombinant dans E. coli.

**9.** Utilisation d'un vecteur d'expression auxiliaire qui contient le gène d'un ARNt qui incorpore l'arginine et reconnaît les codons AGG et/ou AGA pour augmenter le rendement lors de l'expression d'un gène recombinant qui contient le codon AGA et/ou AGG pour l'arginine, dans E. coli par cotransfection de cellules de E. coli avec le vecteur d'expression auxiliaire et un vecteur contenant le gène recombinant, le vecteur d'expression auxiliaire présentant une origine de réplication différente de celle du vecteur contenant le gène recombinant.

Steigerung der Vitalität von rec-t-PA
produzierenden E. coli durch das
Expressionshilfsplasmid pUBS 500